Europäisches Patentamt
European Patent Office
Office européen des brevets

(19)

(11) Veröffentlichungsnummer: **0 036 133**
**A2**

(12)

# EUROPÄISCHE PATENTANMELDUNG

(21) Anmeldenummer: **81101566.8**

(22) Anmeldetag: **05.03.81**

(51) Int. Cl.$^3$: **C 07 D 251/44**
C 09 B 62/04

(30) Priorität: **19.03.80 DE 3010502**

(43) Veröffentlichungstag der Anmeldung:
**23.09.81 Patentblatt 81/38**

(84) Benannte Vertragsstaaten:
**CH DE FR GB IT LI**

(71) Anmelder: **HOECHST AKTIENGESELLSCHAFT**
**Postfach 80 03 20**
**D-6230 Frankfurt/Main 80(DE)**

(72) Erfinder: **Otten, Joachim, Dr.**

**Verstorben(DE)**

(72) Erfinder: **Meininger, Fritz, Dr.**
**Loreleistrasse 7**
**D-6230 Frankfurt am Main 80(DE)**

(54) **Verfahren zur Herstellung von Dihalogentriazinyl-amino-naphthol-Verbindungen.**

(57) Dihalogen-triazinyl-aminonaphthol-Verbindungen der allgemeinen Formel (1)

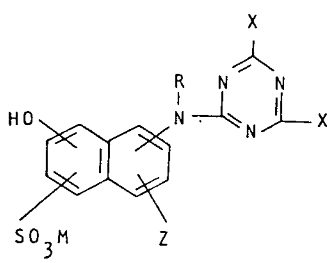

(1)

in welcher die Reste X jeder ein Chlor-, Brom- oder Fluoratom bedeutet, R ein Wasserstoffatom oder eine Alkylgruppe von 1 bis 4 C-Atomen ist, M ein Wasserstoffatom oder das Äquivalent eines Metalls bedeutet und Z für ein Wasserstoffatom oder eine Gruppe der Formel $-SO_3M$ mit M der obengenannten Bedeutung steht, werden durch Umsetzung einer Aminonaphthol-Verbindung der allgemeinen Formel (2)

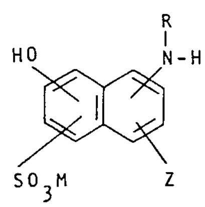

(2)

in welcher R, M und Z die obengenannten Bedeutungen haben, mit einer Cyanurhalogenid-Verbindung der allgemeinen Formel (3)

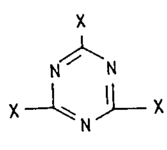

(3)

in welcher X die obengenannten Bedeutungen haben, in Wasser als Reaktionsmedium bei einem pH-Wert von 5 oder kleiner als 5 hergestellt, indem man die Aminonaphtholverbindung der Formel (2) in fester, ungelöster Form in die Reaktion einsetzt und die Umsetzung in Suspension durchführt.

EP 0 036 133 A2

Verfahren zur Herstellung von Dihalogentriazinyl-amino-naphthol-Verbindungen

Die vorliegende Erfindung liegt auf dem technischen Gebiet der Herstellung von Zwischenprodukten, die insbesondere in der Weiterverarbeitung zu faserreaktiven Verbindungen, wie Farbstoffen, eingesetzt werden.

Dihalogentriazinyl-aminonaphthol-Verbindungen sind bekannt. Sie werden durch Acylierung von Aminonaphthol-Verbindungen mittels Cyanurhalogeniden hergestellt. So geht man beispielsweise in der britischen Patentschrift 221 843 in der Weise vor, daß man zuerst Cyanurchlorid in Aceton löst und diese Lösung sodann in Eiswasser verrührt, um eine feinverteilte Suspension von Cyanurchlorid in Wasser zu erhalten. Zu dieser Suspension wird sodann als zweiter Reaktionspartner die Aminonaphtholsulfonsäure in Form einer alkalischen oder neutralen wäßrigen Lösung gegeben.

Diese prinzipielle Arbeitsweise wird auch in neuerer Literatur, wie beispielsweise in W.F. Beech, Fibre-Reactive Dyes (London, 1970), Seite 152, oder in K. Venkataraman, The Chemistry of Synthetic Dyes, Band VI - Reaktive Dyes (New York and London, 1972), Seite 262, beschrieben. Die als Reaktionskomponenten eingesetzten Aminonaphtholsulfonsäuren werden in jedem Falle in Form einer wäßrigen, neutralen oder schwach alkalischen Lösung in die Acylierungsreaktion eingesetzt. -
Auch bei analogen Acylierungsreaktionen, in denen als Cyanurhalogenid Cyanurbromid oder Cyanurfluorid als Reaktionspartner verwendet wird, wird die Aminonaphtholsulfonsäure immer als wäßrige Lösung, die unter Verwendung von Alkali zubereitet wurde, in die Reaktion eingesetzt (vgl. deutsche Offenlegungsschrift 27 47 011, (Beispiel 1).

Die bekannten Verfahren haben den Nachteil, daß wäßrige Lösungen von Aminonaphtholsulfonsäuren im neutralen oder alkalischen Bereich nicht ausreichend stabil sind, da beim Stehen an der Luft offensichtlich Oxidationsreaktionen auftreten, die bei einer nachfolgenden Farbstoffherstellung zu gefärbten Begleitstoffen führen, welche die Nuance und die Echtheit der Färbungen qualitativ vermindern können. Des weiteren treten bei der Acylierungsreaktion unter Verwendung solcher alkalischer oder neutraler Aminonaphtholsulfonsäurelösungen Nebenreaktionen auf, sofern die Acylierung bei pH-Werten oberhalb von 5 erfolgt.

Mit der vorliegenden Erfindung wurde nun ein verbessertes Verfahren zur Herstellung von Dihalogentriazinyl-aminonaphthol-Verbindungen gefunden, das diese Nachteile vermeidet.

Die vorliegende Erfindung betrifft ein Verfahren zur Herstellung von Dihalogentriazinyl-aminonaphthol-Verbindungen der allgemeinen Formel (1)

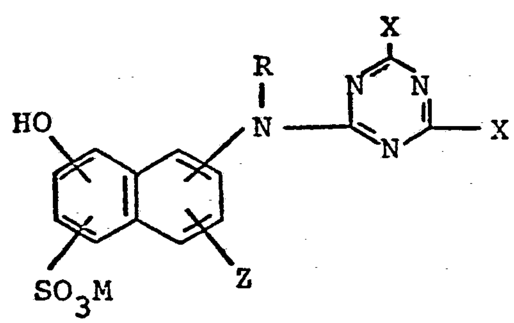

(1)

in welcher die Reste X jeder ein Fluor-, Chlor- oder Bromatom bedeutet, R ein Wasserstoffatom oder eine Alkylgruppe von 1 bis 4 C-Atomen, wie eine Methyl- oder Äthylgruppe, ist, M ein Wasserstoffatom oder das Äquivalent eines Metalls, vorzugsweise eines Alkalimetalls, wie Natrium oder Kalium, bedeutet und Z für ein Wasserstoffatom oder für die Gruppe der Formel $-SO_3M$ mit M der obengenannten Bedeutung steht,
durch Umsetzung einer Aminonaphtholsulfonsäure der

allgemeinen Formel (2)

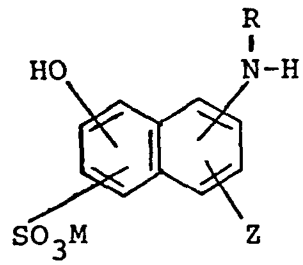

(2)

in welcher R, M und Z die obengenannten Bedeutungen haben, wobei hier M vorzugsweise ein Wasserstoffatom ist, mit einer Cyanurhalogenid-Verbindung der allgemeinen Formel (3)

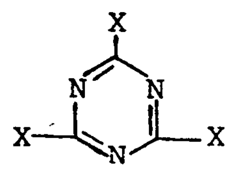

(3)

in welcher X die obengenannte Bedeutung besitzt, in Wasser als Reaktionsmedium bei einem pH-Wert von 5 oder kleiner als 5, vorzugsweise kleiner als 5, das dadurch gekennzeichnet ist, daß man die Aminonaphtholsulfonsäure-Verbindung der allgemeinen Formel (2) in fester, ungelöster Form in die Reaktion einsetzt und daß die Umsetzung in Suspension erfolgt.

Die Umsetzung wird bevorzugt bei einem pH-Wert zwischen 1 und kleiner als 5, insbesondere bevorzugt bei der Verwendung von Cyanurchlorid oder Cyanurbromid bei einem pH-Wert zwischen 1 und 3 und insbesondere bevorzugt bei der Verwendung von Cyanurfluorid als Reaktionspartner bei einem pH-Wert zwischen 3 und kleiner als 5 durchgeführt.

Die Aminonaphtholsulfonsäure-Verbindungen der allgemeinen Formel (2) können in die Reaktion als trockene Substanz, in kristalliner oder bevorzugt gemahlener, pulvriger Form oder in Form einer wäßrigen Suspension oder Aufschlämmung mit einem pH-Wert von 5 oder kleiner als 5 in die Reaktion eingesetzt werden. Trotz des Vorliegens der Reaktionspartner in Suspension tritt die Acylierungsreaktion recht zügig ein, so daß sie in verhältnismäßig kurzer Zeit beendet ist. Es kann bei der erfindungsgemäßen Reaktion auf jeglichen Zusatz von den sonst üblichen Lösungsmitteln, wie beispielsweise Aceton, zum Lösen des Cyanurhalogenids oder auf Lösungsvermittler, wie Emulgatoren, Tenside oder ähnliche Hilfsstoffe, verzichtet werden.

Verbindungen der allgemeinen Formel (2) sind beispielsweise 1-Amino-8-naphthol-3,6-disulfonsäure, 1-Amino-8-naphthol-4,6-disulfonsäure, 2-Amino-5-naphthol-7-sulfonsäure, 2-Methylamino-5-naphthol-7-sulfonsäure, 2-Amino-8-naphthol-6-sulfonsäure und 2-Methylamino-8-naphthol-6-sulfonsäure und deren Alkalimetallsalze, wie die Mono- und Dinatrium-Salze.

Verbindungen der allgemeinen Formel (3) sind 2,4,6-Trichlor-1,3,5-triazin, 2,4,6-Tribrom-1,3,5-triazin und 2,4,6-Trifluor-1,3,5-triazin.

Durch die erfindungsgemäße Arbeitsweise der Herstellung von Dihalogentriazinyl-aminonaphtholsulfonsäuren, die Aminonaphtholsulfonsäure nicht als neutrale oder alkalische wäßrige Lösung, sondern in fester Substanz einzusetzen, ergeben sich gegenüber den bekannten Verfahren deutliche Vorteile: Zum einen wird ein Arbeitsgang eingespart, in dem das Zubereiten der Aminonaphtholsulfonsäurelösung entfällt. Des weiteren wird vermieden, daß Oxidationen der Aminonaphtholsulfonsäuren in den wäßrigen Lösungen, wie oben erwähnt, auftreten. Weiterhin werden mit dem erfindungsgemäßen Verfahren unerwünschte Nebenreaktionen weitgehend unterdrückt, und die Reinheit, Qualität und Ausbeute an den erfindungsgemäß hergestellten Verbindungen der allgemeinen Formel (1) werden im Vergleich zu den bekannten Verfahrensweisen deutlich erhöht; aus diesem Grunde ist es bei dem Verfahren der vorliegenden Erfindung wesentlich, dieses bei einem pH-Wert von 5 oder bevorzugt unterhalb 5 durchzuführen.

Die bei der erfindungsgemäßen Umsetzung frei werdende Halogenwasserstoffsäure wird vorteilhaft durch säurebindende Mittel gebunden, wobei jedoch der verwendete pH-Bereich des Verfahrens nicht verlassen werden darf. Bevorzugt verwendet man schwach-basisch wirkende Verbindungen, wie die Alkalimetallsalze von schwachen Säuren, wie der Essigsäure oder der Kohlensäure, wie beispielsweise Natrium- oder Kaliumcarbonat, Natriumacetat und Natriumbicarbonat, oder auch Dinatriumhydrogenphosphat. Diese alkalischen, insbesondere schwach alkalischen, säurebindenden Mittel können in pulveriger Form durch Einstreuen oder als wäßrige Lösungen während der Reaktionsführung dem Reaktionsansatz zugegeben werden.

Die Durchführung des erfindungsgemäßen Verfahrens erfolgt bei einer Temperatur zwischen -10°C und +30°C, vorzugsweise bei einer Temperatur zwischen -5°C und +20°C. Vorteilhaft soll das erfindungsgemäße Verfahren auch unter kräftiger Durchmischung, wie kräftigem Rühren, durchgeführt werden, um eine schnelle Durchmischung der Reaktanten zu gewährleisten.

Die mit dem erfindungsgemäßen Verfahren erhältlichen Reaktionsgemische, die die Verbindungen der allgemeinen Formel (1) enthalten, können direkt als "Zwischenprodukt" der Weiterverarbeitung zu Farbstoffen oder deren Vorstufen zugeführt werden. So verwendet man diese Reaktionsgemische ohne vorherige Isolierung der Verbindungen der Formel (1), gegebenenfalls nach einer vorherigen Kondensation mit einem (weiteren) aliphatischen oder aromatischen Amin unter Bildung eines sekundären Kondensationsproduktes des Triazins, als Kupplungskomponente für die Herstellung von Azofarbstoffen.

Die nachfolgenden Beispiele dienen zur Erläuterung der Erfindung. Die Teile sind Gewichtsteile, die Prozentangaben beziehen sich auf Gewichtsprozente, sofern nicht anders vermerkt. Gewichtsteile verhalten sich zu Volumenteilen wie Kilogramm zu Liter.

Beispiel 1

Unter Rühren werden 319 Teile 1-Amino-8-naphthol-3,6-disulfonsäure als trockene, gemahlene Ware in eine Suspension aus 190 Teilen feinkristallinem Cyanurchlorid in 1300 Teilen Wasser und 400 Teilen Eis eingetragen; dieses Reaktionsgemisch wird noch 3 Stunden weitergerührt, wobei die Temperatur zwischen 12 und 16°C und der pH-Wert mittels etwa 160 Teilen Natriumbicarbonat, das portionsweise in pulveriger Form eingestreut wird, bei 1,7 bis 2,3 gehalten werden. Nach dieser Zeit ist die Umsetzung beendet und kein freies Amin mehr nachweisbar.

Eine Probe der Reaktionslösung wird mit einer beliebigen Diazoniumsalzlösung, beispielsweise mit einer frisch bereiteten Lösung von diazotiertem Anilin, in üblicher Weise titrimetrisch gekuppelt. Es wird eine Ausbeute von 95 - 98 % d.Th. an der erfindungsgemäß erhaltenen 1-(4',6'-Dichlor-1',3',5'-triazin-2'-yl)-amino-8-naphthol-3,6-disulfonsäure ermittelt.

Die so hergestellte Dichlortriazinyl-aminonaphtholsulfonsäure kann direkt zur Herstellung eines Azofarbstoffes eingesetzt werden.

Beispiel 2

Zu einer Suspension aus 190 Teilen Cyanurchlorid in 1000 Teilen Wasser und 700 Teilen Eis werden innerhalb einer halben Stunde 319 Teile 1-Amino-8-naphthol-4,6-disulfonsäure in Form eines trocknen Pulvers eingetragen. Man hält die Temperatur durch Zugabe von Eis im Bereich zwischen 0°C und +5°C. Der pH-Wert wird durch tropfenweise Zugabe einer gesättigten wäßrigen Natriumhydrogencarbonatlösung bei einem pH-Wert zwischen 1,5 und 2,0 gehalten. Der Reaktionsansatz wird sodann noch kräftig weitergerührt. Nach etwa 3 Stunden ist kein freies Amin

mehr nachweisbar.

Die Ausbeute an 1-(4',6'-Dichlor-1',3',5'-triazin-2'-yl)-amino-8-naphthol-4,6-disulfonsäure wird gemäß einer Titration mit einer Diazoniumsalzlösung mit etwa 98 % d.Th. bestimmt.

Die aus der Synthese erhaltene Lösung bzw. Suspension mit der Dichlortriazinyl-aminonaphtholsulfonsäure als Kupplungskomponente kann+) direkt zur Herstellung von Azofarbstoffen einsetzen. +) man

Beispiel 3

239 Teile 2-Amino-5-naphthol-7-sulfonsäure werden in trockener, gemahlener Form in 5000 Teilen Wasser suspendiert. Unter kräftigem Rühren werden 190 Teile Cyanurchlorid in kristalliner Form hinzugegeben, wobei man eine Temperatur zwischen 5 und 10°C einhält. Man rührt diesen Reaktionsansatz noch 3 Stunden lang bei dieser Temperatur nach und hält hierbei durch Einstreuen von Natriumbicarbonat den pH-Wert auf 2,5 bis 3. Nach dieser Zeit ist keine Ausgangsverbindung mehr nachweisbar.

Die so hergestellte 2-(4',6'-Dichlor-1',3',5'-triazin-2'-yl)-amino-5-naphthol-7-sulfonsäure wird in einer Ausbeute von über 95 % d.Th. erhalten.

Beispiel 4

Man verfährt wie im Beispiel 3 beschrieben, setzt jedoch als Aminonaphtholsulfonsäure 253 Teile 2-Methylamino-8-naphthol-6-sulfonsäure ein. Das entstandene Reaktionsgemisch, das die 2-(4',6'-Dichlor-1',3',5'-triazin-2'-yl)-methylamino-8-naphthol-6-sulfonsäure in hoher Ausbeute und Reinheit enthält, kann direkt zur Herstellung von Azofarbstoffen verwendet werden.

Beispiel 5

Man verfährt in gleicher Weise, wie im Beispiel 1 beschrieben, verwendet jedoch als Acylierungsmittel 320 Teile Cyanurbromid. Das Reaktionsgemisch enthält in gleich guter Ausbeute und Reinheit die 1-(4',6'-Dibrom-1',3',5'-triazin-2'-yl)-amino-8-naphthol-3,6-disulfonsäure.

Beispiel 6

Zu einer Suspension aus 319 Teilen gemahlener 1-Amino-8-naphthol-3,6-disulfonsäure in 4000 Teilen Eiswasser werden unter kräftigem Rühren 135 Teile 2,4,6-Trifluor-1,3,5-triazin bei 0°C zugetropft. Durch langsames Einstreuen von pulverförmigem Natriumbicarbonat wird der pH-Wert zwischen 3,5 und 4,0 gehalten. Nach 30 Minuten ist die Reaktion beendet. Die titrimetrisch bestimmte Ausbeute an 1-(4',6'-Difluor-1',3',5'-triazin-2'-yl)-amino-8-naphthol-3,6-disulfonsäure beträgt über 95 % d.Th.

Beispiel 7

Eine Suspension von 319 Teilen pulverförmiger 1-Amino-8-naphthol-4,6-disulfonsäure in 3000 Teilen Eiswasser werden bei 0°C unter Rühren langsam mit 140 Teilen 2,4,6-Trifluor-1,3,5-triazin versetzt. Durch langsame Zugabe einer gesättigten wäßrigen Lösung von Natriumbicarbonat von 0°C wird ein pH-Wert von 3,5 bis 4,0 eingestellt. Danach wird das Gemisch noch 20 bis 30 Minuten lang gerührt, bis keine Ausgangsverbindung mehr nachweisbar ist.

Durch titrimetrische Bestimmung mittels einer Diazoniumsalzlösung wird eine Ausbeute von über 95 % d.Th. an 1-(4',6'-Difluor-1',3',5'-triazin-2'-yl)-amino-8-naphthol-4,6-disulfonsäure ermittelt.

Beispiel 8

239 Teile 2-Amino-5-naphthol-7-sulfonsäure werden in

pulveriger Form in 6000 Teilen Eiswasser suspendiert. Unter kräftigem Rühren werden 140 Teile 2,4,6-Trifluor-1,3,5-triazin zugetropft, wobei die Temperatur bei 0°C gehalten wird. Durch allmähliches Einstreuen von Natriumbicarbonat wird ein pH-Wert zwischen 3,5 und 4,0 eingestellt und gehalten. Bei diesem pH-Wert läßt man den Reaktionsansatz noch 20 bis 30 Minuten nachrühren, bis keine Ausgangsverbindung mehr nachweisbar ist.

In diesem Reaktionsgemisch ist die 2-(4',6'-Difluor-1',3',5'-triazin-2'-yl)-amino-5-naphthol-7-sulfonsäure in einer Ausbeute von 95 - 98 % d.Th. enthalten.

Die aus der Synthese erhaltene wäßrige Lösung bzw. Suspension dieser Difluor-triazinyl-aminonaphtholsulfonsäure kann direkt zur Herstellung von Farbstoffen eingesetzt werden.

Beispiel 9

Eine Suspension von 254 Teilen pulverförmiger 2-Methylamino-8-naphthol-6-sulfonsäure in 6000 Teilen Eiswasser wird langsam mit 140 Teilen 2,4,6-Trifluor-1,3,5-triazin versetzt, wobei die Temperatur bei 0°C und der pH-Wert bei 3,5 bis 4,0 mittels Einstreuen von Natriumacetat gehalten wird. Unter weiterem kräftigem Rühren wird der Reaktionsansatz noch etwa 30 Minuten lang bei 0°C und einem pH-Wert von 3,5 bis 4,0 nachgerührt, bis kein freies Amin mehr nachweisbar ist. In dem entstandenen Gemisch ist die 2-(4',6'-Difluor-1',3',5'-triazin-2'-yl)-methylamino-8-naphthol-6-sulfonsäure in einer Ausbeute von über 95 % d.Th. enthalten.

Beispiel 10

Die gemäß Beispiel 1 nach dem erfindungsgemäßen Verfahren hergestellte Lösung bzw. Suspension der Dichlortriazinylamino-naphthol-disulfonsäure kann wie folgt weiterver-

arbeitet werden: Zur Durchführung einer Kupplungsreaktion zu einem Azofarbstoff wird sie in üblicher Weise mit einer salzsauren, wäßrigen Diazoniumsalzlösung aus 173 Teilen Anilin-2-sulfonsäure, die in üblicher Weise hergestellt worden war, versetzt; hierbei wird die Kupplung bei einem pH-Wert von 4,5 bis 5,0 durchgeführt, der zunächst durch Einstreuen von 15 Teilen Natriumdihydrogenphosphat und sodann durch Einstreuen von Natriumbicarbonat gehalten wird. Die Kupplung wird bei 15 bis 18°C durchgeführt. Nachdem keine Diazoniumverbindung durch eine Tüpfelprobe mehr nachweisbar ist, fügt man zu dieser Azoverbindung eine Lösung von 300 Teilen 4-ß-Sulfatoäthylsulfonyl-anilin und 105 Teilen Natriumbicarbonat in 1000 Teilen Wasser hinzu. Dieses Reaktionsgemisch wird bei einem pH-Wert von 5,5 bis 6,0 zunächst eine Stunde lang bei 25 - 30°C gerührt; sodann wird die Reaktionstemperatur innerhalb von 2 Stunden langsam auf 50°C erhöht. Der gebildete Farbstoff wird mit Kaliumchlorid ausgefällt und isoliert. Man erhält den aus der deutschen Patentschrift 1 265 698 bekannten Farbstoff der Formel

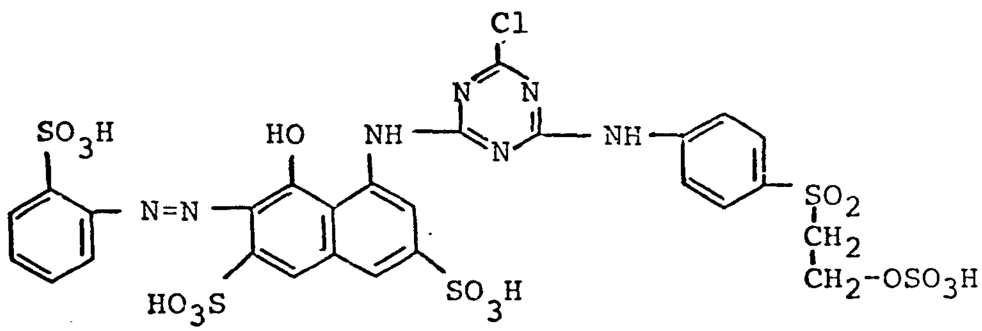

als Kalium/Natriumsalz. - Bezogen auf die eingesetzte Aminonaphtholdisulfonsäure wird dieser Farbstoff im Vergleich zu dem in der deutschen Patentschrift 1 265 698 beschriebenen Verfahren+) erhalten. Außerdem weist der Farbstoff, der aus dem erfindungsgemäß hergestellten Vorprodukt erhalten wird, in der Färbung auf Wolle ein wesentlich besseres Naßechtheitsniveau auf, vor allem eine bessere Waschechtheit bei 60°C und eine deutliche Überlegenheit in der alkalischen Schweißechtheit.

+) in einer 10 % höheren Ausbeute

PATENTANSPRÜCHE:

1. Verfahren zur Herstellung von Dihalogen-triazinyl-aminonaphthol-Verbindungen der allgemeinen Formel (1)

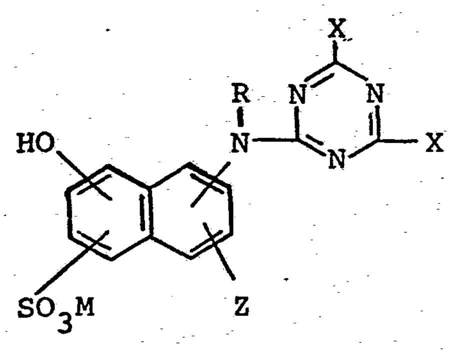

(1)

in welcher die Reste X jeder ein Chlor-, Brom- oder Fluoratom bedeutet, R ein Wasserstoffatom oder eine Alkylgruppe von 1 bis 4 C-Atomen ist, M ein Wasserstoffatom oder das Äquivalent eines Metalls bedeutet und Z für ein Wasserstoffatom oder eine Gruppe der Formel $-SO_3M$ mit M der obengenannten Bedeutung steht, durch Umsetzung einer Aminonaphthol-Verbindung der allgemeinen Formel (2)

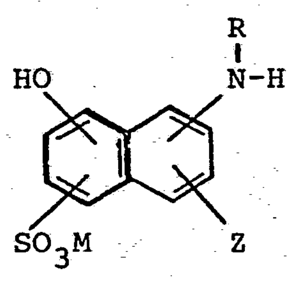

(2)

in welcher R, M und Z die obengenannten Bedeutungen haben, M hier jedoch bevorzugt ein Wasserstoffatom ist, mit einer Cyanurhalogenid-Verbindung der allgemeinen Formel (3)

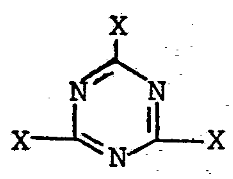

(3)

in welcher X die oben genannten Bedeutungen haben, in

Wasser als Reaktionsmedium bei einem pH-Wert von 5 oder kleiner als 5, dadurch gekennzeichnet, daß man die Aminonaphtholverbindung der allgemeinen Formel (2) in fester, ungelöster Form in die Reaktion einsetzt und daß die Umsetzung in Suspension erfolgt.

2. Verfahren nach Anspruch 1, dadurch gekennzeichnet, daß man die Umsetzung bei einem pH-Wert von 1 bis kleiner als 5 durchführt.

3. Verfahren nach Anspruch 1, dadurch gekennzeichnet, daß man die Umsetzung mit 2,4,6-Trichlor-1,3,5-triazin bei einem pH-Wert von 1 bis 3 durchführt.

4. Verfahren nach Anspruch 1, dadurch gekennzeichnet, daß man die Umsetzung mit 2,4,6-Trifluor-1,3,5-triazin bei einem pH-Wert von 3 bis kleiner als 5 durchführt.